# EUROPEAN PATENT SPECIFICATION

(11) **EP 3 429 517 B1**
(45) Date of publication and mention of the grant of the patent: **08.04.2026**
(21) Application number: 17767402.5
(22) Date of filing: 15.03.2017
(51) Int. Cl.: A61F 7/00, A61B 17/132, A61B 18/02, A61B 5/01, A61F 7/10, A61B 5/026, A61F 7/02, A61B 17/135, A61B 17/00

(54) **COOLING SLEEVE AND TOURNIQUET**
KÜHLMANSCHETTE UND STAUBINDE
MANCHON DE REFROIDISSEMENT ET GARROT

(30) Priority: 15.03.2016 US 201662308425 P
(43) Date of publication of application: 23.01.2019
(73) Proprietor: University of Washington, Seattle, WA 98195 (US)
(72) Inventor: AARABI, Shahram, Seattle WA 98195 (US)
(74) Representative: MacLean, Martin Robert
(86) International application number: PCT/US2017/022442
(87) International publication number: WO 2017/160934

(56) References cited:
- WO-A1-2015/051457
- WO-A1-2015/106317
- WO-A1-2015/106317
- WO-A1-2017/035573
- US-A- 5 407 421
- US-A- 5 871 526
- US-A1- 2008 234 788
- US-A1- 2008 234 788
- US-A1- 2010 324 429
- US-A1- 2012 065 561
- US-A1- 2012 065 561
- US-A1- 2012 130 457
- US-A1- 2013 090 683
- US-A1- 2015 297 909
- US-A1- 2015 351 957
- US-B2- 7 691 084
- MABRY ROBERT L.: "Tourniquet Use on the Battlefield", MILITARY MEDICINE, vol. 171, no. 5, 1 May 2006 (2006-05-01), US, pages 352 - 356, XP055907694, ISSN: 0026-4075, Retrieved from the Internet <URL:https://watermark.silverchair.com/milmed.171.5.352.pdf?token=AQECAHi208BE49Ooan9kkhW_Ercy7Dm3ZL_9Cf3qfKAc485ysgAAAuowggLmBgkqhkiG9w0BBwagggLXMIIC0wIBADCCAswGCSqGSIb3DQEHATAeBglghkgBZQMEAS4wEQQMNcIis7QfXdx4hETKAgEQgIICnR82G22KcgljoqM5fA-MFDcpcNsg58VFZWu8nF6pt2X8ru-uijJTx2EOMEuvQkv1ASLEyDRGrRoDCIzYdbW> [retrieved on 20220331], DOI: 10.7205/MILMED.171.5.352
- WANG SHOUWEN: "Pneumatic Tourniquet for Surgical Procedures of Hemodialysis Vascular Access", SEMINARS IN DIALYSIS, vol. 28, no. 1, 27 March 2014 (2014-03-27), US, pages 81 - 89, XP055907705, ISSN: 0894-0959, Retrieved from the Internet <URL:https://api.wiley.com/onlinelibrary/tdm/v1/articles/10.1111%2Fsdi.12242> [retrieved on 20220331], DOI: 10.1111/sdi.12242

## Description

### BACKGROUND

Acute limb ischemia (ALI) is sudden interruption of limb perfusion and can be caused by limb trauma, atherosclerosis, or life-saving hemostatic therapies such as tourniquets or resuscitative endovascular balloon occlusion of the aorta (REBOA). As used herein, ALL is defined to include the loss of perfusion to an extremity from any cause, including a loss of blood flow due to the application of a tourniquet. In four years of recent war in Iraq and Afghanistan, limb threatening extremity injuries cost the military $40 million in immediate costs plus $1 billion in lifetime disability costs. Every year in the United States, ALI affects 200,000 people and leads to 40,000 amputated arms and legs at a cost to the healthcare system of $7 billion, as well as a human cost impossible to quantify.

Therapeutic cooling has been shown to prevent ischemic damage in the laboratory and in organ transplantation, neurologic surgery, and cardiac surgery by reducing tissue and organ metabolic rate. Cooling is used sporadically by surgeons to preserve ischemic limbs in the field and during transport but this has not become standard of care due to logistical difficulties in providing sustained cooling to a limb *in situ.* A therapeutic limb cooling system is disclosed as a solution to potentially rescue life and limb in traumatic acute limb ischemia. It is believed that, in a swine model, experimental limb cooling to 5-15°C would improve local limb muscle tissue metabolism during ALI compared to uncooled limbs.

Joint Theater Trauma Registry data from 2006-2011 revealed that 14% of casualties with major vascular injury who underwent damage control resuscitation (DCR) and vascular repair still required delayed amputation. These casualties were evacuated quickly with fasciotomy and vascular shunting to decrease ischemic limb injury. Fasciotomy, rapid transport, and temporary shunting are not adequate to prevent amputation in casualties with ALI receiving DCR. Further, a large number of patients go on to suffer permanent disability from muscle and nerve injury despite successful limb salvage.

A rise in asymmetric warfare and terrorist attacks in military settings, as well as civilian (accidental) trauma-inducing events have led directly to an increase in the occurrence of ALI. Tourniquet use has experienced a resurgence following recent military experience that demonstrates that tourniquets save lives. This practice has reached the policy level of mandating tourniquet availability and training in the civilian setting. After the Sandy Hook school shooting, the Hartford Consensus was formed and the Executive Branch and FEMA enacted policy to promote hemorrhage control training among the public including, specifically, tourniquet use. These policies are reaching the state level. However, limbs are still being lost, perhaps as a consequence of tourniquet use itself. The possibility of extending the time which a tourniquet can be used safely is an unmet need of trauma, military, and pre-hospital medicine. Similarly, REBOA is gaining popularity in the civilian and military settings. However, the primary complication of REBOA use is ALI, with the rate of limb loss associated with REBOA being as high as 12% in some series. Further, in addition to traumatic and iatrogenic causes of ALI, atherosclerosis is the primary cause of ALI overall and is a chronic disease of aging. With the rapid surge in our elderly civilian population - termed by some as the 'silver tsunami' - we can expect a continuing increase in non-traumatic ALI cases and limb loss.

Recent studies, including animal studies, suggest that the period prior to nerve and other tissue damage can be extended, and/or nerve damage can be minimized, if the limb and, in particular, the tissue region including the nerve is rapidly and significantly cooled. Because of the low thermal conductivities of human tissue, however, rapid core limb cooling can be difficult to achieve. To provide core (deep) limb cooling rapidly it is necessary to lower skin temperatures about the ischemic limb. However, subzero skin temperatures can lead to tissue injury and destruction, most notably frostbite or cold burn. Moreover, at sufficiently cold surface temperatures, typically near or below 0°C, blood vessels close to the skin will constrict. Therefore, the body's own protective mechanisms may hinder rapid core limb cooling.

The present inventor has obtained animal study data suggesting that external cooling to 5-15°C favorably affects limb metabolism during acute limb ischemia in a controlled pig model. Armed with this data and using computer heat transfer models, a novel tourniquet device has been developed that may be used to provide therapeutic cooling to human lower limbs in the event of ischemia. The ischemia may be induced by the device itself, for example, to preserve life in traumatic injury scenarios, or may result from other causes such as a blood clot, or the like. This foundational work will enable rapid translation of limb cooling as a viable new approach to limb salvage during ALI.

As noted earlier, ALI affects approximately 200,000 people in the United States annually, leading to approximately 40,000 amputated arms and legs. When ALI occurs, rapid transfer to a tertiary care facility and restoration of blood flow to the extremity by a vascular surgeon is the only treatment. Every minute without blood flow increases the likelihood of amputation or death. Therefore rapid surgical revascularization is imperative to avoid permanent tissue damage and loss of limb. Improvements to ALI individual outcomes may be improved if ischemic tissue damage, and in particular nerve damage, can be delayed or reduced prior to revascularization US2008/0234788A1 describes a grounded pressure cooling apparatus. WO 2015/106317 A1 describes a multifunctional brace for rehabilitation and for post-operative use in orthopaedic surgery. US 2013/090683 A1 describes a system for maintaining or altering the body temperature. US 20120065561 A1 describes a compression device for applying compression to an extremity of a mammal. US 20150297909 A1 describes an apparatus for assisting with the physiological recovery from fatigue, stress or injury. Mabry (Mil Med. 2006 May;171(5):352-6) describes tourniquet use on the battlefield. Wang (Semin Dial. 2015 Jan-Feb;28(1):81-9) describes pneumatic tourniquet for surgical procedures of hemodialysis vascular access. US 5871526 A1 describes a portable temperature control system.

### SUMMARY

The present invention is defined by the claims.

The invention provides a cooling tourniquet system as defined by claim 1.

A cooling tourniquet system includes a cooling component having a cooling pad and a cover member. The cooling pad is configured to be wrapped about a limb of a body with a contact surface for placement in contact with the limb. The cover member encloses the cooling pad and includes a tourniquet on a proximal portion of the cover member. The temperature of the contact surface is controllable during use, and includes one or more temperature sensors that are configured to detect a temperature of the limb and to generate corresponding limb temperature data. A control component is operably connected to receive the limb temperature data from the at least one temperature sensor, and configured to control the temperature of the contact surface based on the received limb temperature data. For example, the control component may control the temperature of the contact surface to achieve a rapid deep cooling of the limb of the body without causing any frostbite or other cold-related tissue damage.

In some embodiments a plurality of temperature sensors are provided and spaced apart on the contact surface of the cooling pad.

In some embodiments a distal portion of the cover member has an air bladder configured to urge the cooling pad against the limb during use.

In some embodiments the cover member includes a display operatively connected to the control component and configured to display a status of the cooling tourniquet system.

### DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 illustrates a cooling sleeve and tourniquet, shown for application to a human leg;
FIGURE 2 illustrates an exploded view of the cooling sleeve and tourniquet shown in FIGURE 1;
FIGURE 3 illustrates a cover with an integrated tourniquet for the cooling sleeve and tourniquet shown in FIGURE 1;
FIGURE 4 illustrates a liquid bladder assembly for the cooling sleeve and tourniquet shown in FIGURE 1; and
FIGURE 5 is a system diagram for the cooling sleeve and tourniquet shown in FIGURE 1.

### DETAILED DESCRIPTION

Although acute limb ischemia (ALI) is a treatable condition, delayed treatment can result in permanent disability, amputation, or even death to the victim. The standard of care for ALI is surgical intervention. The inventors are unaware of any pre-surgical intervention options to improve outcomes related to ALI. As discussed above, in some instances
ALI may be induced to prevent loss of life or limb. For example, in the event of an injury to a limb causing a rapid loss of blood, a tourniquet may be applied proximally from the injury to control venous and arterial circulation to the injured limb for a period of time. Sufficient pressure is applied to cause the underlying vessels to occlude. For example, tourniquets are frequently required for in-field treatment of injuries to military personnel.

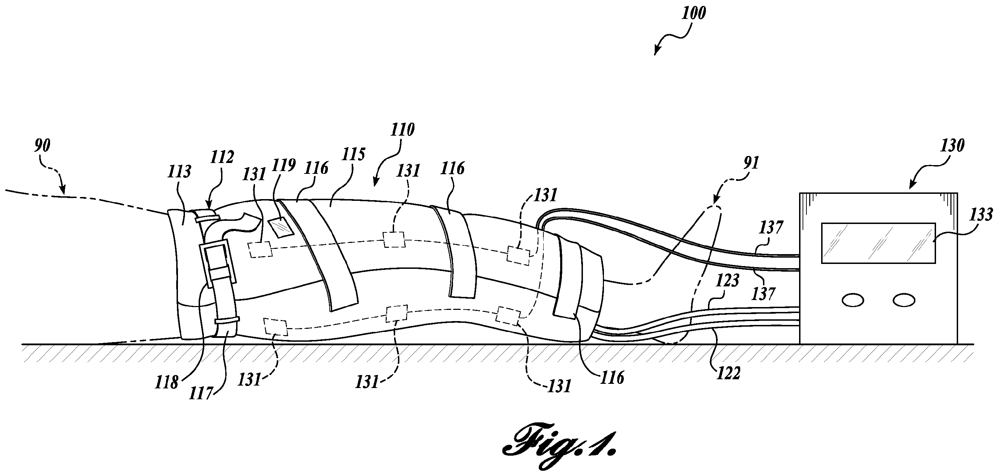

A novel tourniquet system is disclosed that prevents or reduces tissue damage from ALI, whether the ALI results from internal vessel blockages or from the application of a tourniquet to prevent life-threatening loss of blood. The present invention is defined by the claims. An embodiment of a cooling sleeve and tourniquet system 100 in accordance with the present invention is shown in FIGURE 1 applied to a leg or limb 91 of an individual 90. For example, the tourniquet system 100 may be used to treat soldier or civilian victims of an explosive device in the field prior to transporting the victim to a medical facility. The system 100 may also be used (without applying the tourniquet) for a victim of ALI resulting from a non-traumatic cause, for example an embolism, during transportation to a hospital.

The cooling device and tourniquet system 100 in this embodiment includes a multi-part cooling/tourniquet component 110 configured to be wrapped around the limb 91, and a control component 130 that monitors and controls the temperature applied by the cooling/tourniquet component 110 in order to produce rapid deep cooling. In particular, the system 100 is configured to cool the injured limb 91 at least to a depth that includes the major nerves in the limb 91 as rapidly as possible, without causing frostbite or other tissue damage to the limb 91.

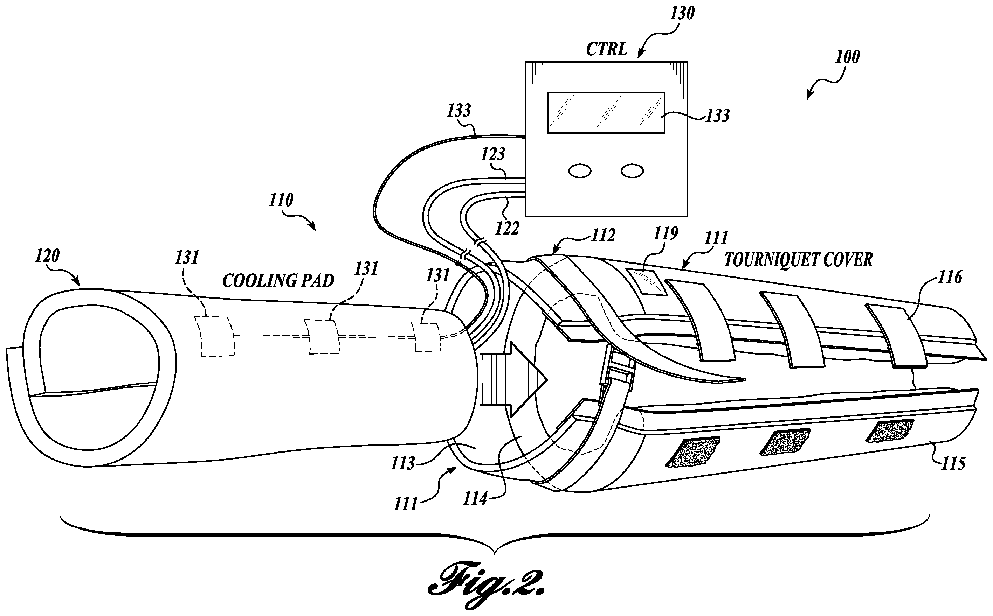

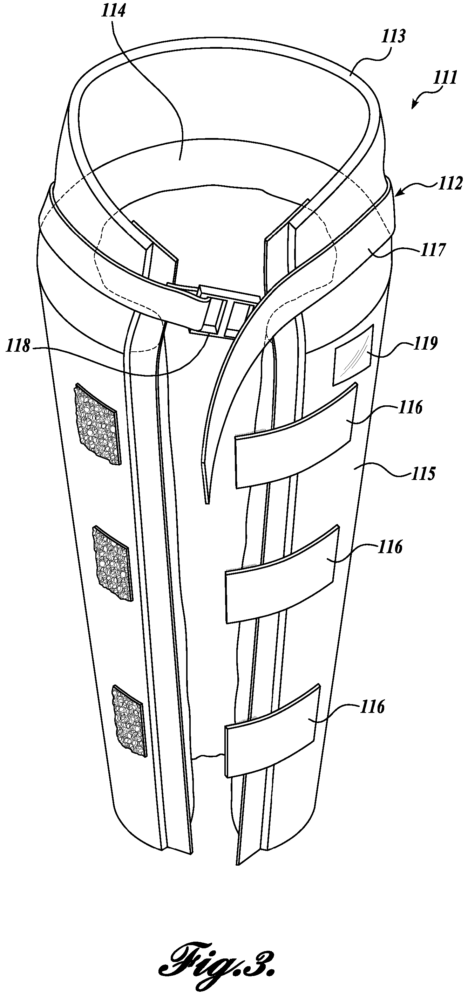

Refer now also to FIGURE 2, which illustrates the cooling/tourniquet component 110 with a cover portion 111 separated from a cooling pad 120. Refer also to FIGURE 3, which shows the cover portion 111 in isolation. The cover portion 111 includes a relatively thin proximal sleeve portion 113 and a relatively thick distal portion 115. The proximal sleeve portion 113 includes an integrated tourniquet 112, for example, a flexible strap 117 and an adjustable securement mechanism 118. Although a manual tourniquet 112 is illustrated, other types of tourniquets are contemplated and may alternatively be used. For example, a pneumatic tourniquet may be used.

The tourniquet 112 is configured to be tightened about the limb 91 with sufficient pressure to occlude blood flow to the limb 91, and to maintain the pressure until loosened by the caregiver. The distal portion 115 includes an inner support device, for example, an air bladder 114. The support device 114 is configured to provide a supporting pressure to the limb 91, pressing the cooling pad 120 against the limb 91 during use. In some embodiments the bladder 114 is operatively connected to an inflator (not shown) such that the bladder 114 may be inflated to a desired pressure after securing the component 110 to the limb. The bladder 114 also provides the benefit of essentially immobilizing or stabilizing the limb 91. Other methods of stabilizing the limb may be used. In some embodiments the bladder 114 may be provided external of the cover portion 111. Alternatively or additionally, a system of relatively rigid bars in an insulating foam may be used. Optionally, the cover portion 111 may include a display 119, to display data regarding the system 100. For example, the display 119 may display a skin temperature of the limb 91, and/or a time since the system 100 was engaged, or the like.

A plurality of securement straps 116 close the cover portion 111 and secure the cooling pad 120 around the limb 91. Other securement mechanisms are known and may be used. For example, the cover portion 111 may be provided with a zipper or hook-and-loop fastening means along a length of the cover 111 for closing the cover portion 111 about the limb 91.

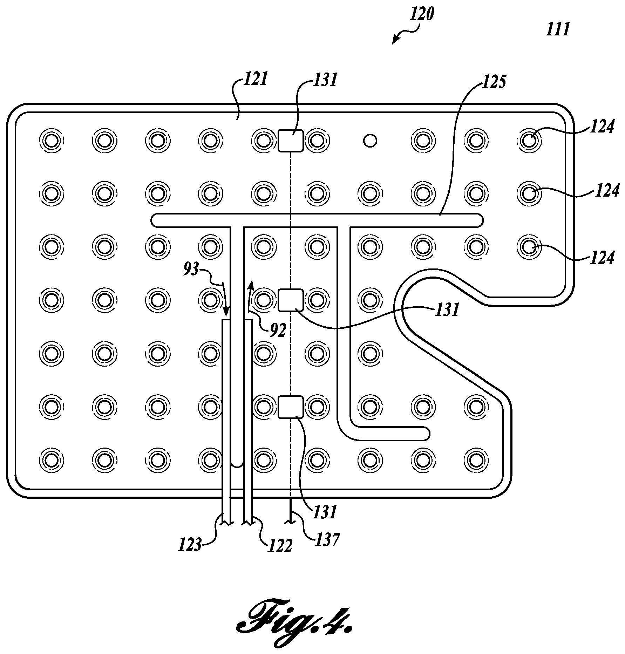

Refer now also to FIGURE 4, which is a plan view of the cooling pad 120. The cooling pad 120 in this embodiment comprises a fluid bladder 121 having an inlet port 122 and an outlet port 123. An array of attachment points 124 (for example, heat welds) connect opposite sides of the fluid bladder 121 such that opposite walls of the fluid bladder 121 are joined, thereby limiting the thickness of the bladder 121 under pressure. Elongate heat welds 125 provide flow blockages in the fluid bladder 121, to define a general flow path through the fluid bladder 121 between the inlet port 122 and the outlet port 123. For example, in this embodiment fluid entering through the inlet port 122 (indicated by arrow 92) must travel around the elongate heat welds 125 to return to the outlet port 123 (indicated by arrow 93). A plurality of temperature sensors 131 are fixed to an outer surface of the cooling pad 120, and positioned to engage the limb 91 when the system 100 is in use. A means for transmitting the data from the sensors 131 is provided, for example, a wireless system, a signal wire 137, or the like.

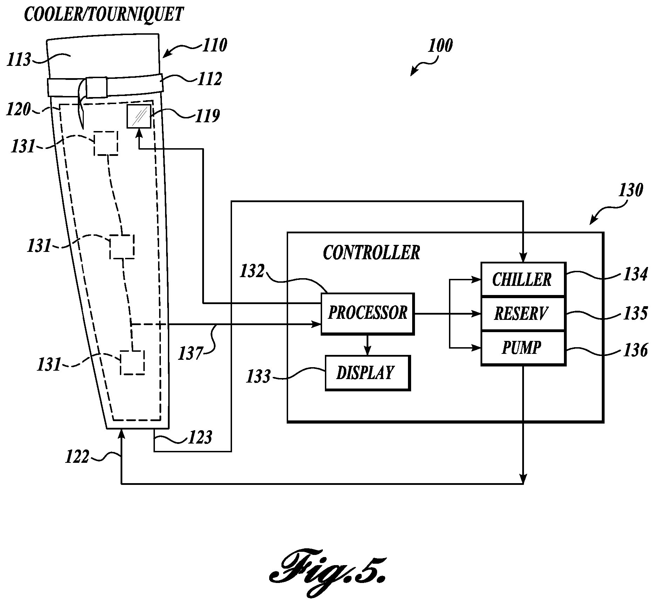

Refer now to FIGURES 1 and 5. FIGURE 5 illustrates in diagram the system 100, including components of the control component 130. The controller component 130 includes a processor 132 that is in signal communication with one or more sensors 131, for example, temperature sensors configured to detect a temperature at or near the skin of the limb 91, and/or pressure sensors configured to detect a pressure in the fluid bladder 121 or in the air bladder 114. The processor 132 may optionally drive a display 133 that displays information regarding the status of the system 100. For example, the display 133 may be configured to display one or more of a temperature of the limb 91, a temperature of the cooling fluid, a flow rate of the cooling fluid, a time showing how long the system 100 has been in continuous operation, a countdown timer, a pressure of the bladders 114, 121, or the like. Additionally or alternatively, the processor 132 may control the display 119 (if present) on the cooling/tourniquet component 110.

The control system 130 in this embodiment further includes a fluid chiller 134, a coolant reservoir 135, and a pump 136. The processor 132 receives data from the sensors 131, and uses the received data to operate the chiller 134 and the pump 136, to provide a controllable coolant flow rate and coolant flow temperature to the inlet port 122 of the cooling pad 120. Optionally, the control system 130 may also be operatively connected 138 to the display 119 on the cover portion 111, for example, to display data regarding the status of the system 100.

For example, in order to cool the injured limb 91 as rapidly as possible, the control system 130 may initially produce a cooling fluid flow through the cooling pad 120 at a relatively high flow rate and/or at a relatively low temperature. For example, the coolant temperature may initially be below zero degrees Celsius. As the temperature of the injured limb 91 decreases (based on data from the sensors 131, the flow rate and/or the cooling fluid temperature may be moderated, for example to between 5 °C and 15 °C, to avoid causing tissue damage. It will also be appreciated that the inner support device 114 of the cooling component 120 (for example, the air bladder) urges the cooling pad 120 firmly against the limb 91, to ensure good thermal contact is maintained to facilitate heat transfer, and to provide accurate sensor data to the controller component 130.

As discussed above, in order to prevent or moderate tissue damage, and in particular nerve damage, is it desirable to provide deep cooling of the limb 91 as rapidly as possible.

To use the system 100 the cooling pad 120 is placed around the limb, with a surface of the cooling pad in contact with the limb. It is contemplated that a thin fabric (not shown), for example a thin cotton fabric wrap or sleeve, may be provided between the limb and the contact surface of the cooling pad 120. Such an arrangement is expressly defined to be encompassed by the contact surface of the cooling pad being in contact with, or adjacent, the limb. In some embodiments the thin wrap or sleeve may be wet, for example by soaking in water. The cooling pad 120 sensors 131 detect a temperature of the limb adjacent the cooling pad 120. The cover portion 111 supports the cooling pad 120 against the limb. If indicated by the situation, for example if a significant loss of blood is occurring, the tourniquet 112 on the relatively flexible portion of the cover portion 111 is tensioned to stop the distal flow of blood. The control component 130 controls the temperature of the cooling pad 120, e.g., by controlling the fluid temperature to the cooling pad 120, based on temperature data from the sensors 131, to produce a time-varying temperature to the limb to accelerate the cooling of the limb without causing damage to limb tissue.

Although a currently preferred embodiment of the cooling and tourniquet system has been described, other embodiments are contemplated. For example, in another embodiment an array of thermoelectric cooling (TEC) elements are provided in a flexible wrap that is applied to the injured limb 91. The TEC elements are controlled by the control component, based on sensor feedback from the injured limb 91, to provide controlled cooling to limb 91. It will be appreciated that the array of TEC elements provide the advantage that the cooling may be more precisely controlled. In particular, the TEC elements may be individually controlled, so that the cooling temperature applied to the limb may vary along the surface of the limb.

Similarly, it is contemplated that the cooling pad 120 may have a plurality of independently controlled sections. For example, the cooling pad may have two or more fluidly separated sections, each with separate inlet and outlet ports, and the control component may be configured to provide flows to each section based on sensor data received from the portion of the limb corresponding to each section, such that the cooling is provided zonally.

## Claims

1. A cooling tourniquet system (100) for treatment of traumatic limb injury, the system comprising:
a cooling component (110) having a cooling pad (120) configured to be wrapped about a limb of a body with a contact surface for placement in contact with the limb (91), and a cover member (111) comprising a proximal portion (113) and a distal portion (115) with regard to the flow of arterial blood, the cover member (111) configured to enclose the cooling pad (120) wrapped about the limb (91), wherein a temperature of the contact surface is controllable during use, and further comprising at least one temperature sensor (131) on the contact surface that is configured to detect a temperature of the limb (91) and to generate corresponding limb temperature data;
wherein the cooling pad (120) comprises a fluid bladder (121) having an inlet port (122) and an outlet port (123);
a control component (130) configured to control a temperature of a coolant fluid flowing into the inlet port (122), the control component comprising a processor (132) operably connected to receive the limb temperature data from the at least one temperature sensor (131), a reservoir (135) of the coolant fluid, a chiller (134) configured to modify the temperature of the coolant fluid in the reservoir, and a pump (136) configured to pump the coolant fluid from the reservoir (135) to the inlet port (122);
wherein the cover member further comprises a tourniquet (112) supported on the proximal portion of the cooling component (110), the tourniquet (112) configured to be selectively applied to stop blood flow to the limb distally from the tourniquet (112), and the control component is configured to control the temperature of the contact surface based on the received limb temperature data;
**characterised in that** the cooling pad (120) comprises a plurality of zones, wherein the control component (130) is configured to control a respective contact surface temperature of each of the plurality of zones.

2. The cooling tourniquet system (100) of Claim 1, wherein the at least one temperature sensor (131) on the contact surface of the cooling pad (120) comprises a plurality of spaced-apart temperature sensors (131) disposed on the contact surface of the cooling pad (120).

3. The cooling tourniquet system (100) of Claim 1, wherein the cover member (111) further comprises a display (119) operatively connected to the control component (130) and configured to display a status of the cooling tourniquet system (100).

## Patentansprüche

1. Kühlstaubindensystem (100) zur Behandlung einer traumatischen Extremitätenverletzung, wobei das System umfasst:
eine Kühlkomponente (110), aufweisend ein Kühlkissen (120), das das dazu konfiguriert ist, um eine Extremität eines Körpers mit einer Kontaktfläche zur Platzierung in Kontakt mit der Extremität (91) gewickelt zu werden, und ein Abdeckungselement (111), umfassend einen proximalen Abschnitt (113) und einen distalen Abschnitt (115) in Bezug auf den Fluss von arteriellem Blut, wobei das Abdeckungselement (111) dazu konfiguriert ist, das Kühlkissen (120), das um die Extremität (91) gewickelt ist, zu umschließen, wobei eine Temperatur der Kontaktfläche im Gebrauch steuerbar ist, und ferner umfassend mindestens einen Temperatursensor (131) auf der Kontaktfläche, der dazu konfiguriert ist, eine Temperatur der Extremität (91) zu erfassen und entsprechende Extremitätentemperaturdaten zu erzeugen;
wobei das Kühlkissen (120) eine Fluidblase (121) umfasst, aufweisend eine Einlassöffnung (122) und eine Auslassöffnung (123);
eine Steuerkomponente (130), die dazu konfiguriert ist, eine Temperatur eines Kühlfluids, das in die Einlassöffnung (122) fließt, zu steuern, wobei die Steuerkomponente einen Prozessor (132), der betriebsfähig verbunden ist, um die Extremitätentemperaturdaten von dem mindestens einen Temperatursensor (131) zu empfangen, ein Reservoir (135) des Kühlfluids, ein Kühlaggregat (134), das dazu konfiguriert ist, die Temperatur des Kühlfluids in dem Reservoir zu modifizieren, und eine Pumpe (136), die dazu konfiguriert ist, das Kühlfluid aus dem Reservoir (135) zu der Einlassöffnung (122) zu pumpen, umfasst;
wobei das Abdeckungselement ferner eine Staubinde (112) umfasst, die auf dem proximalen Abschnitt der Kühlkomponente (110) getragen wird, wobei die Staubinde (112) dazu konfiguriert ist, selektiv angewendet zu werden, um einen Blutfluss zu der Extremität distal von der Staubinde (112) zu stoppen, und die Steuerkomponente dazu konfiguriert ist, die Temperatur der Kontaktfläche auf der Basis der empfangenen Extremitätentemperaturdaten zu steuern;
**dadurch gekennzeichnet, dass** das Kühlkissen (120) eine Vielzahl von Zonen umfasst, wobei die Steuerkomponente (130) dazu konfiguriert ist, eine jeweilige Kontaktflächentemperatur jeder der Vielzahl von Zonen zu steuern.

2. Kühlstaubindensystem (100) nach Anspruch 1, wobei der mindestens eine Temperatursensor (131) auf der Kontaktfläche des Kühlkissens (120) eine Vielzahl von beabstandeten Temperatursensoren (131) umfasst, die auf der Kontaktfläche des Kühlkissens (120) angeordnet ist.

3. Kühlstaubindensystem (100) nach Anspruch 1, wobei das Abdeckungselement (111) ferner eine Anzeige (119) umfasst, die funktionsfähig mit der Steuerkomponente (130) verbunden ist und dazu konfiguriert ist, einen Status des Kühlstaubindensystems (100) anzuzeigen.

## Revendications

1. Système de garrot de refroidissement (100) pour le traitement d'une blessure traumatique d'un membre, le système comprenant :
un composant de refroidissement (110) ayant un coussin de refroidissement (120) configuré pour être enroulé autour d'un membre d'un corps avec une surface de contact pour un placement en contact avec le membre (91), et un élément de couverture (111) comprenant une partie proximale (113) et une partie distale (115) par rapport au flux de sang artériel, l'élément de couverture (111) configuré pour enfermer le coussin de refroidissement (120) enroulé autour du membre (91), dans lequel une température de la surface de contact peut être commandée pendant l'utilisation, et comprenant en outre au moins un capteur de température (131) sur la surface de contact qui est configuré pour détecter une température du membre (91) et pour générer des données de température de membre correspondantes ;
dans lequel le coussin de refroidissement (120) comprend une vessie de fluide (121) ayant un orifice d'entrée (122) et un orifice de sortie (123) ;
un composant de commande (130) configuré pour commander une température d'un fluide de refroidissement s'écoulant jusque dans l'orifice d'entrée (122), le composant de commande comprenant un processeur (132) connecté de manière fonctionnelle pour recevoir les données de température de membre de l'au moins un capteur de température (131), un réservoir (135) du fluide de refroidissement, un refroidisseur (134) configuré pour modifier la température du fluide de refroidissement dans le réservoir, et une pompe (136) configurée pour pomper le fluide de refroidissement du réservoir (135) à l'orifice d'entrée (122) ;
dans lequel l'élément de couverture comprend en outre un garrot (112) supporté sur la partie proximale du composant de refroidissement (110), le garrot (112) étant configuré pour être appliqué sélectivement pour arrêter le flux sanguin vers le membre de manière distale par rapport au garrot (112), et le composant de commande est configuré pour commander la température de la surface de contact sur la base des données de température de membre reçues ;
**caractérisé en ce que** le coussin de refroidissement (120) comprend une pluralité de zones, dans lequel le composant de commande (130) est configuré pour commander une température de surface de contact respective de chacune de la pluralité de zones.

2. Système de garrot de refroidissement (100) selon la revendication 1, dans lequel l'au moins un capteur de température (131) sur la surface de contact du coussin de refroidissement (120) comprend une pluralité de capteurs de température (131) espacés les uns des autres disposés sur la surface de contact du coussin de refroidissement (120).

3. Système de garrot de refroidissement (100) selon la revendication 1, dans lequel l'élément de couverture (111) comprend en outre un dispositif d'affichage (119) connecté de manière fonctionnelle au composant de commande (130) et configuré pour afficher un état du système de garrot de refroidissement (100).
